# EUROPEAN PATENT APPLICATION

(11) **EP 4 779 302 A1**
(43) Date of publication of application: **22.07.2026**
(21) Application number: 25876072.7
(22) Date of filing: 06.11.2025
(51) Int. Cl.: G01N 33/00, G01N 1/14

(54) **RADIOACTIVE GAS DETECTION DEVICE AND DETECTION METHOD**

(30) Priority: 25.11.2024 CN 202411695176
(71) Applicant: China Nuclear Power Technology Research Institute Co., Ltd., Shenzhen, Guangdong 518031 (CN)
(72) Inventor: LI, Shiying, Shenzhen, Guangdong 518031 (CN); DU, Yingzhe, Shenzhen, Guangdong 518031 (CN); DAI, Genggeng, Shenzhen, Guangdong 518031 (CN); LIU, Xiaohua, Shenzhen, Guangdong 518031 (CN); XIAO, Chaoyuan, Shenzhen, Guangdong 518031 (CN); CHANG, Kang, Shenzhen, Guangdong 518031 (CN); LIN, Peng, Shenzhen, Guangdong 518031 (CN); SHAN, Chenyu, Shenzhen, Guangdong 518031 (CN); LIU, Xiajie, Shenzhen, Guangdong 518031 (CN)
(74) Representative: Curell Suñol S.L.P.
(86) International application number: PCT/CN2025/133069
(87) International publication number: WO 2026/108626

(57) **Abstract**

A radioactive gas detection device and method are provided. The radioactive gas detection device is used for detecting a radioactive gas containing a target radionuclide, and includes an adsorption assembly, a sampling assembly, a monitoring assembly, a desorption assembly, a detection assembly, a first control valve assembly, and a controller. The sampling assembly, the adsorption assembly, and the monitoring assembly are in communication in sequence. The adsorption assembly is further in communication with the detection assembly. The controller is configured to: in response to the monitoring assembly detecting that the target radionuclide is contained in the radioactive gas that has passed through the adsorption assembly, control the first control valve assembly to disconnect the adsorption assembly from the sampling assembly, disconnect the adsorption assembly from the monitoring assembly, and communicate the adsorption assembly with the detection assembly, and control the detection assembly to perform a detection operation on the target radionuclide released from the adsorption assembly to obtain the content of the target radionuclide. The radioactive gas detection device can improve the detection accuracy of the target radionuclide in the radioactive gas.

## Description

### TECHNICAL FIELD

The present disclosure relates to the field of radioactive gas detection, and in particular, to a radioactive gas detection device and method.

### BACKGROUND

Gas emission standards are typically formulated for industries that generate exhaust gas during production to prevent environmental pollution and ensure sustainable development. For example, in the nuclear power industry, airborne radioactive effluents need to be purified, and their total activity, activity concentration, and the composition of key radionuclides must be measured. Only after confirming compliance with regulatory limits may the effluents be released into the atmosphere through a chimney.

In related technologies, when a detector is used to detect trace radionuclides, an adsorbent material containing trace radionuclides, such as filter paper, is often placed on a detection window of the detector. The activity concentration of α/β particles released from the trace radionuclides is measured using α/β characteristic track analysis to detect the concentration of trace radioactive inert gas radionuclides. However, it is found during the detection process that when the filter paper or other adsorbent material is placed directly in the detection window of the detector, only a part of the particles entering the detection window can be detected, while others escaping into the environment cannot be detected, resulting in poor detection accuracy. Such detectors perform adequately when the target radionuclide is at a preset concentration but exhibit significantly reduced accuracy when the radionuclide content is too low..

### SUMMARY

The present disclosure aims to address at least one of technical problems in the existing technology. To this end, the present disclosure provides a radioactive gas detection device that enhances the detection accuracy of a target radionuclide in a radioactive gas.

The present disclosure further provides a detection method using the device.

According to a first aspect of the present disclosure, an embodiment provides a radioactive gas detection device for detecting a radioactive gas containing a target radionuclide, including:
an adsorption assembly, configured to adsorb the target radionuclide from the radioactive gas;
a sampling assembly, in communication with the adsorption assembly and configured to supply the radioactive gas to the adsorption assembly;
a monitoring assembly, in communication with the adsorption assembly and configured to monitor the target radionuclide in the radioactive gas that has passed through the adsorption assembly;
a desorption assembly, configured to cause the adsorption assembly to release the adsorbed target radionuclide;
a detection assembly, in communication with the adsorption assembly and configured to measure a content of the target radionuclide released from the adsorption assembly;
a first control valve assembly, configured to control communication between the adsorption assembly and the sampling assembly, between the adsorption assembly and the monitoring assembly, and between the adsorption assembly and the detection assembly; and
a controller, where the controller is configured to: in response to the monitoring assembly detecting that the target radionuclide is contained in the radioactive gas that has passed through the adsorption assembly, control the first control valve assembly to disconnect the adsorption assembly from the sampling assembly, disconnect the adsorption assembly from the monitoring assembly, and communicate the adsorption assembly with the detection assembly, and control the detection assembly to perform a detection operation on the target radionuclide released from the adsorption assembly to obtain the content of the target radionuclide.

The radioactive gas detection device according to the embodiments in the first aspect of the present disclosure at least has the following beneficial effects.
1. The adsorption assembly adsorbs the target radionuclide from the radioactive gas. When the adsorption assembly is unable to completely absorb the target radionuclides in the radioactive gas, the monitoring assembly detects the presence of the target radionuclide in the radioactive gas that has passed through the adsorption assembly. In this case, the controller controls the first control valve assembly to disconnect the adsorption assembly from the sampling assembly, disconnect the adsorption assembly from the monitoring assembly, and communicate the adsorption assembly with the detection assembly. The controller further controls the desorption assembly to cause the adsorption assembly to release the adsorbed target radionuclide, and controls the detection assembly to detect the content of the target radionuclide released from the adsorption assembly. As such, the detection accuracy of the target radionuclide is improved.
2. The monitoring assembly monitors the change in the concentration of the radioactive gas passing through the adsorption assembly, and determines whether the adsorption assembly has completed adsorption. This allows the adsorption assembly to sufficiently adsorb the target radionuclides, with only a small amount of the target radionuclides escaping, thereby minimizing the impact of the escaped target radionuclides on the detection accuracy of the target radionuclide. In addition, the sufficient amount of target radionuclides can meet detection requirements of the detection assembly, enabling more accurate detection by the detection assembly.

According to some embodiments of the present disclosure, the radioactive gas detection device further includes a transfer assembly configured to transfer the target radionuclide released from the adsorption assembly to the detection assembly.

According to some embodiments of the present disclosure, the detection assembly includes: an equipment chamber, a detection chamber, a partition membrane, a partition plate, and a detection component, where the equipment chamber and the detection chamber are in communication with each other through an incidence window, and the detection chamber is in communication with the adsorption assembly; the partition membrane is arranged at the incidence window; the partition plate is arranged at the incidence window and is located on a side of the partition membrane away from the equipment chamber, and the partition plate is configured to block or expose the incidence window; the detection component is arranged in the equipment chamber;
where the controller is configured to: control the partition plate to block the incidence window, in response to the transfer assembly transferring the target radionuclide released from the adsorption assembly to the detection chamber; and control the partition plate to expose the incidence window and control the detection component in the equipment chamber to perform the detection operation, in response to a transfer operation having been completed for the adsorption assembly.

According to some embodiments of the present disclosure, the detection assembly includes: a detection chamber, a detection component, a working component, and a working valve, where the detection chamber is in communication with the adsorption assembly, the detection component is arranged in the detection chamber, the working component is in communication with the detection chamber and is configured to introduce a working gas into the detection chamber, and the working valve is configured to control communication between the working component and the detection chamber;
where the controller is configured to: in response to the transfer assembly performing a transfer operation or in response to a transfer operation having been completed for the adsorption assembly, control the working valve to communicate the working component with the detection chamber, control the working component to introduce the working gas into the detection chamber, and control the detection component in the detection chamber to perform the detection operation.

According to some embodiments of the present disclosure, the transfer assembly, the adsorption assembly, and the detection assembly are in communication in sequence, and the transfer assembly is configured to deliver a purge gas that does not react with the target radionuclide to the detection assembly, so as to purge the target radionuclide released from the adsorption assembly to the detection assembly; and/or
the transfer assembly includes a vacuumizing component and a second control valve assembly, the vacuumizing component is in communication with the detection assembly, and the second control valve assembly is configured to control communication between the detection assembly and the vacuumizing component and communication between the detection assembly and the adsorption assembly;
where the controller is configured to: in response to the transfer assembly performing a transfer operation, control the second control valve assembly to communicate the detection assembly with the vacuumizing component and disconnect the detection assembly from the adsorption assembly, and control the vacuumizing component to perform a vacuumizing operation; and in response to a preset negative pressure being formed in the detection assembly, control the second control valve assembly to disconnect the detection assembly from the vacuumizing component and communicate the detection assembly with the adsorption assembly.

According to some embodiments of the present disclosure, the sampling assembly includes a gas container and a gas flow meter, the gas container is in communication with the adsorption assembly and is configured to store the radioactive gas, and the gas flow meter is arranged in a pipeline between the gas container and the adsorption assembly and is configured to measure a total amount of the radioactive gas entering the adsorption assembly; or
the sampling assembly includes a to-be-tested pipeline, a sample inlet pipeline, a sample outlet pipeline, and a gas flow meter, the to-be-tested pipeline is configured to discharge the radioactive gas and has a first position and a second position distributed in sequence along a discharge direction, the sample inlet pipeline is in communication with the to-be-tested pipeline and the adsorption assembly when the to-be-tested pipeline is at the first position, the sample outlet pipeline is in communication with the to-be-tested pipeline and the monitoring assembly when the to-be-tested pipeline is at the second position, and the gas flow meter is arranged in the sample inlet pipeline and is configured to measure a total amount of the radioactive gas entering the adsorption assembly.

According to some embodiments of the present disclosure, the adsorption assembly includes a housing and a porous adsorption member, an adsorption chamber is defined by the housing, the adsorption chamber has a first communication position and a second communication position, the housing is in communication with the sampling assembly when the adsorption chamber is at the first communication position, the housing is in communication with the monitoring assembly or the detection assembly when the adsorption chamber is at the second communication position, and the porous adsorption member is arranged in the adsorption chamber and is arranged between the first communication position and the second communication position along a delivery direction of the radioactive gas; or
the adsorption assembly includes a housing and a separation membrane, a first chamber and a second chamber are defined by the housing, the first chamber is in communication with the sampling assembly, the second chamber is in communication with the monitoring assembly or the detection assembly, and the separation membrane is arranged between the first chamber and the second chamber and is in communication with the first chamber and the second chamber.

According to some embodiments of the present disclosure, the adsorption assembly includes a housing and a separation membrane, and a pressure in the second chamber is lower than a pressure in the first chamber.

According to a second aspect of the present disclosure, an embodiment provides a detection method, including:
S1: delivering a radioactive gas to pass through an adsorption assembly, allowing the adsorption assembly to adsorb a target radionuclide from the radioactive gas;
S2: in response to a monitoring assembly detecting that the target radionuclide is contained in the radioactive gas that has passed through the adsorption assembly, ending the delivery of the radioactive gas and acquiring a total amount X₁ of the delivered radioactive gas and a content X₂ of the target radionuclide adsorbed by the adsorption assembly; and
S3: acquiring a concentration of the target radionuclide according to the total amount X₁ of the radioactive gas and the content X₂ of the target radionuclide.

The detection method according to the embodiments in the second aspect of the present disclosure has at least the following beneficial effects. By using the monitoring assembly to monitor whether the radioactive gas that has passed through the adsorption assembly contains the target radionuclide, it can be determined whether the adsorption assembly has completed adsorption. This enables the adsorption assembly to adsorb a sufficient amount of target radionuclides for detection, while allowing fewer target radionuclides to pass through the monitoring assembly. As a result, the detection accuracy and the content accuracy of the target radionuclide are improved, thereby enhancing the detection accuracy of the concentration of the target radionuclide in the radioactive gas.

According to some embodiments of the present disclosure, in S2, heating is performed to release the target radionuclide adsorbed by the adsorption assembly, and the content X₂ of the target radionuclide released from the adsorption assembly is measured, where a temperature of the heating ranges from 80°C to 100°C.

Additional aspects and advantages of the present disclosure will be partly given in and partly apparent from the description below, or understood through practice of the present disclosure.

### BRIEF DESCRIPTION OF DRAWINGS

The present disclosure will be further described below with reference to the accompanying drawings and embodiments. In the drawings:
FIG. 1 is a schematic structural diagram showing sampling from a gas container by a sampling assembly in a radioactive gas detection device according to an embodiment of the present disclosure;
FIG. 2 is a schematic structural diagram showing sampling from a to-be-tested pipeline by a sampling assembly in a radioactive gas detection device according to an embodiment of the present disclosure;
FIG. 3 is a schematic structural diagram of a radioactive gas detection device according to an embodiment of the present disclosure, where the adsorption assembly includes a porous adsorption member;
FIG. 4 is a schematic structural diagram of a radioactive gas detection device according to an embodiment of the present disclosure, where the adsorption assembly includes a separation membrane and purge transfer is used;
FIG. 5 is a schematic structural diagram of a radioactive gas detection device according to an embodiment of the present disclosure, where the detection assembly is a windowed detector and negative pressure transfer is used; and
FIG. 6 is a schematic structural diagram of a radioactive gas detection device according to an embodiment of the present disclosure, where the detection assembly is a windowless detector and negative pressure transfer is used.

List of reference numerals:
adsorption assembly 100; housing 110; first chamber 111; second chamber 112; porous adsorption member 120; separation membrane 130; back pressure valve 140;
sampling assembly 200; gas container 210; gas flow meter 220; to-be-tested pipeline 230; sample inlet pipeline 240; sample outlet pipeline 250;
monitoring assembly 300;
desorption assembly 400;
detection assembly 500; equipment chamber 510; detection chamber 520; partition membrane 530; partition plate 540; detection component 550; working component 560; working valve 570;
first control valve assembly 600; first valve 610; second valve 620; third valve 630;
transfer assembly 700; vacuumizing component 710; second control valve assembly 720.

### DETAILED DESCRIPTION

Embodiments of the present disclosure will be described in detail hereinafter in conjunction with accompanying drawings in which the same or like reference characters refer to the same or like elements or elements having the same or like functions throughout. The embodiments described below by reference to the accompanying drawings are illustrative and are intended for illustrating only and are not to be construed as limiting the present disclosure.

In the description of the present disclosure, it should be understood that for the description of orientations, the orientation or positional relationships indicated by the terms such as "on" and "below" are based on orientation or positional relationships shown in the accompanying drawings, and are used only for ease and brevity of illustration and description, rather than indicating or implying that the mentioned apparatus or element must have a particular orientation or must be constructed and operated in a particular orientation. Therefore, such terms should not be construed as limiting of the present disclosure.

In the description of the present disclosure, the term "several" means one or more, and "multiple" and "a plurality of" mean two or more. If used herein, the terms such as "first", "second" and the like are merely used for distinguishing technical features, and are not intended to indicate or imply relative importance, or implicitly point out the number of the indicated technical features, or implicitly point out the order of the indicated technical features.

In the description of the present disclosure, unless otherwise explicitly defined, the terms such as "configure", "install/mount" and "connect" should be understood in a broad sense, and those having ordinary skills in the art can reasonably determine the specific meanings of the above terms in the present disclosure based on the specific contents of the technical scheme.

In related technologies, when a detector is used to detect trace radionuclides, an adsorbent material containing trace radionuclides, such as filter paper, is often placed on a detection window of the detector. The activity concentration of α/β particles released from the trace radionuclides is measured using α/β characteristic track analysis to detect the concentration of trace radioactive inert gas radionuclides. However, it is found during the detection process that when the filter paper or other adsorbent material is placed directly in the detection window of the detector, only the particles perpendicularly entering the detection window can be detected, while others escaping into the environment cannot be detected, resulting in poor detection accuracy. Such detectors perform adequately when the target radionuclide is at a preset concentration but exhibit significantly reduced accuracy when the radionuclide content is too low.

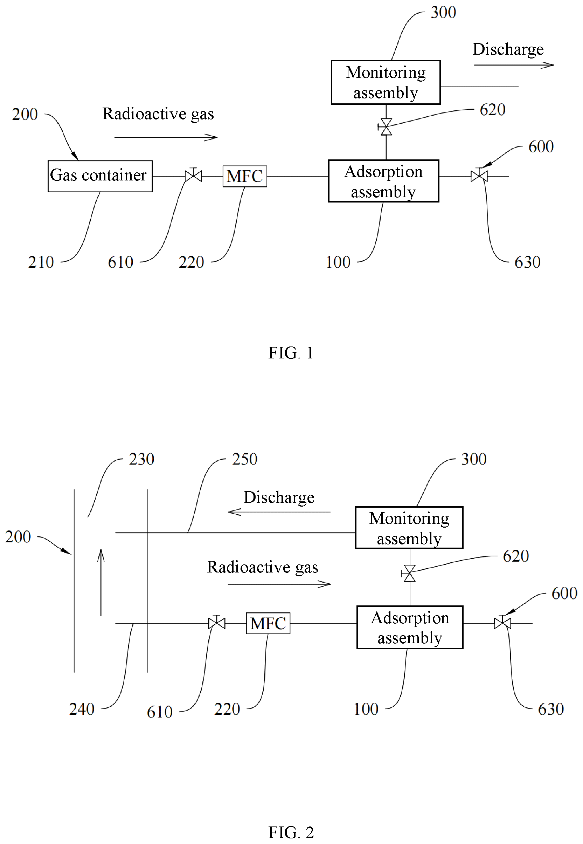

Referring to FIG. 1 to FIG. 6, according to a first aspect of the present disclosure, an embodiment provides a radioactive gas detection device for detecting a radioactive gas containing a target radionuclide. The radioactive gas detection device includes an adsorption assembly 100, a sampling assembly 200, a monitoring assembly 300, a desorption assembly 400, a detection assembly 500, a first control valve assembly 600, and a controller. The sampling assembly 200, the adsorption assembly 100, and the monitoring assembly 300 are in communication in sequence. The adsorption assembly 100 is further in communication with the detection assembly 500.

It should be noted that the radioactive gas refers to a gas containing one or more radionuclides, and the radionuclide refers to an atom that exhibits radioactivity. The target radionuclide refers to one or more radionuclides in the radioactive gas.

In this embodiment, the adsorption assembly 100 is configured to adsorb the target radionuclide from the radioactive gas. For example, the adsorption assembly 100 may contain a porous material, and pore structures in the porous material form a highly ordered network structure, providing a large specific surface area and a suitable adsorption and storage space, thus enhancing the adsorption capacity of the porous material for radionuclide molecules. Moreover, the porous material can selectively adsorb and store the desired radionuclide, so that the radionuclide is separated from other molecules. In other words, the pores of the porous material are dimensioned to precisely accommodate the target radionuclide. When the radioactive gas passes through the porous material of the adsorption assembly 100, the target radionuclide is embedded in the pores of the porous material, while the remaining gas components pass through the porous material. As such, the target radionuclide is effectively extracted from the radioactive gas.

The sampling assembly 200 is in communication with the adsorption assembly 100 and is configured to supply the radioactive gas to the adsorption assembly 100. The radioactive gas is stably supplied by the sampling assembly 200, so that the radioactive gas passes through the adsorption assembly 100 stably, allowing the target radionuclide to be continuously embedded in the pores of the porous material.

The monitoring assembly 300 is in communication with the adsorption assembly 100 and is configured to monitor the target radionuclide in the radioactive gas that has passed through the adsorption assembly 100. The monitoring assembly 300 can monitor in real time whether the target radionuclide is present in the radioactive gas. The monitoring assembly 300 may be implemented as a mass spectrometer, a chromatograph, etc. When the monitoring assembly 300 detects the presence of the target radionuclide in the radioactive gas that has passed through the adsorption assembly 100, it indicates that the adsorption assembly 100 has reached its adsorption capacity.

It should be noted that the adsorption assembly 100 includes an unsaturated state, a substantially saturated state, and a fully saturated state when adsorbing the target radionuclide.

The unsaturated state refers to a condition in which sufficient pores remain available in the porous material after the adsorption assembly 100 has adsorbed all the target radionuclides in the radioactive gas passing therethrough. In the unsaturated state, the monitoring assembly 300 cannot detect the presence of the target radionuclide, and cannot determine the content of the target radionuclide adsorbed by the adsorption assembly 100. If detection is performed while the adsorption assembly 100 is in the unsaturated state, the content of the target radionuclide may be too low, resulting in reduced detection accuracy of the detection assembly 500.

The substantially saturated state refers to a condition in which the adsorption assembly 100 has adsorbed most of the target radionuclides in the radioactive gas passing therethrough and only a small part of the radionuclides passes through the adsorption assembly 100. In the substantially saturated state, the monitoring assembly 300 detects the presence of the target radionuclide, and observes a continuous increase in the content of the target radionuclide. When the monitoring assembly 300 detects the presence of the target radionuclide, the adsorption assembly 100 has just entered the substantially saturated state. At this moment, the content of the target radionuclide in the adsorption assembly 100 is sufficient to the accuracy requirements of the detection assembly 500, while only a minimal amount of the target radionuclides is lost, thereby ensuring higher detection accuracy for the target radionuclide.

The saturated state refers to a condition in which all the pores of the porous material in the adsorption assembly 100 are embedded with the target radionuclide, such that no more target radionuclide can be adsorbed when the radioactive gas passes through the porous material. In the saturated state, the content of the target radionuclide detected by the monitoring assembly 300 remains stable. However, in a period from the moment at which the monitoring assembly 300 detects the presence of the target radionuclide to the moment at which the content of the target radionuclide becomes stable, a part of the target radionuclides pass through the monitoring assembly 300 and are discharged, resulting in a reduction in the content of the target radionuclide and seriously affecting the detection accuracy.

Therefore, in this embodiment, the monitoring assembly 300 monitors whether the radioactive gas passing through the adsorption assembly 100 contains a radionuclide. When detecting the presence of the radionuclide, it is determined that the adsorption assembly 100 has just entered the substantially saturated state. In this case, the adsorption assembly 100 adsorbs an appropriate amount of target radionuclides, which is sufficient for the detection assembly 500 to perform measurements within its accuracy range, thereby achieving higher detection accuracy. In addition, because the target radionuclide is detected by the monitoring assembly 300 immediately when it passes through the monitoring assembly 300, the amount of the target radionuclide escaping therefrom is reduced, and a more accurate content of the target radionuclide can be obtained, improving both the content accuracy of the target radionuclide and the detection accuracy. Consequently, the accuracy of the calculated concentration of the target radionuclide in the radioactive gas can be improved.

The desorption assembly 400 is configured to cause the adsorption assembly 100 to release the adsorbed target radionuclide. For example, the adsorption assembly 100 adsorbs the target radionuclides by embedding them in the pores. When the desorption assembly 400 heats the target radionuclides, the porous material expands thermally. This further enlarges the pores, thereby preventing them from continuing to confine the target radionuclides. As a result, the target radionuclides detach from the pores, and are thereby separated from the porous material. In other words, the adsorption assembly 100 releases the adsorbed target radionuclide under the heating of the desorption assembly 400. Further, the desorption assembly 400 may directly heat the adsorption assembly 100. Alternatively, the porous material may be manually removed from the adsorption assembly 100 and heated separately.

The detection assembly 500 is in communication with the adsorption assembly 100 and is configured to measure a content of the target radionuclide released from the adsorption assembly 100. For example, the detection assembly 500 has a detection surface. The detection assembly 500 detects the target radionuclide through a separate cavity. The target radionuclide in the cavity can emit particles in a plurality of directions. Some of these particles are emitted toward the detection surface, either perpendicularly or obliquely. When the target radionuclide is dispersed in the cavity of the detection assembly, the detection surface can detect particles emitted in different directions in the cavity, so that more particles can be detected and higher detection accuracy of the detection assembly 500 can be achieved.

The first control valve assembly 600 is configured to control communication between the adsorption assembly 100 and the sampling assembly 200, between the adsorption assembly 100 and the monitoring assembly 300, and between the adsorption assembly 100 and the detection assembly 500. The first control valve assembly 600 includes a first valve 610 arranged between the adsorption assembly 100 and the sampling assembly 200, a second valve 620 arranged between the adsorption assembly 100 and the monitoring assembly 300, and a third valve 630 arranged between the adsorption assembly 100 and the detection assembly 500. The second valve 620 may also be arranged at a tail end of the monitoring assembly 300 along a delivery direction of the radioactive gas. In addition, the second valve 620 and the third valve 630 may be integrated as a three-way valve, and the communication between the adsorption assembly 100 and the sampling assembly 200 and between the adsorption assembly 100 and the detection assembly 500 are controlled by the three-way valve.

The controller is electrically connected to the first valve 610, the second valve 620, and the third valve 630 to control opening and closing of the first valve 610, the second valve 620, and the third valve 630. The controller is configured to: in response to the monitoring assembly 300 detecting that the target radionuclide is contained in the radioactive gas that has passed through the adsorption assembly 100, control the first control valve assembly 600 to disconnect the adsorption assembly 100 from the sampling assembly 200, disconnect the adsorption assembly 100 from the monitoring assembly 300. For example, when the monitoring assembly 300 detects that the radioactive gas that has passed through the adsorption assembly 100 contains the target radionuclide, the adsorption assembly 100 is in the substantially saturated state. In this case, the controller controls to close the first valve 610 and the second valve 620, the sampling assembly 200 no longer delivers the radioactive gas to the adsorption assembly 100. As a result, the total amount of the radioactive gas delivered can be determined, and the radioactive gas no longer flows out of the monitoring assembly 300, thus avoiding loss of the target radionuclide. Moreover, the controller is further configured to control the first control valve assembly 600 to communicate the adsorption assembly 100 with the detection assembly 500, and control the detection assembly 500 to perform a detection operation on the target radionuclide released from the adsorption assembly 100 to obtain the content of the target radionuclide. A concentration of the target radionuclide in the radioactive gas can be calculated according to the content of the target radionuclide and the total amount of the radioactive gas.

It should be noted that the adsorption assembly 100 adsorbs the target radionuclide from the radioactive gas. When the adsorption assembly 100 is unable to completely absorb the target radionuclides in the radioactive gas, i.e., when the adsorption assembly 100 reaches the substantially saturated state, the monitoring assembly 300 detects the presence of the target radionuclide in the radioactive gas that has passed through the adsorption assembly 100. In this case, the controller controls the first control valve assembly 600 to disconnect the adsorption assembly 100 from the sampling assembly 200, disconnect the adsorption assembly 100 from the monitoring assembly 300, and communicate the adsorption assembly 100 with the detection assembly 500. The controller further controls the desorption assembly 400 to cause the adsorption assembly 100 to release the adsorbed target radionuclide, and controls the detection assembly 500 to detect the content of the target radionuclide released from the adsorption assembly 100. As such, the detection accuracy of the target radionuclide is improved. The monitoring assembly 300 monitors the change in the concentration of the radioactive gas passing through the adsorption assembly 100, and determines whether the adsorption assembly 100 has completed adsorption. This allows the adsorption assembly 100 to sufficiently adsorb the target radionuclides, with only a small amount of the target radionuclides escaping, thereby minimizing the impact of the escaped target radionuclides on the detection accuracy of the target radionuclide. In addition, the sufficient amount of target radionuclides can meet detection requirements of the detection assembly 500, enabling more accurate detection by the detection assembly 500.

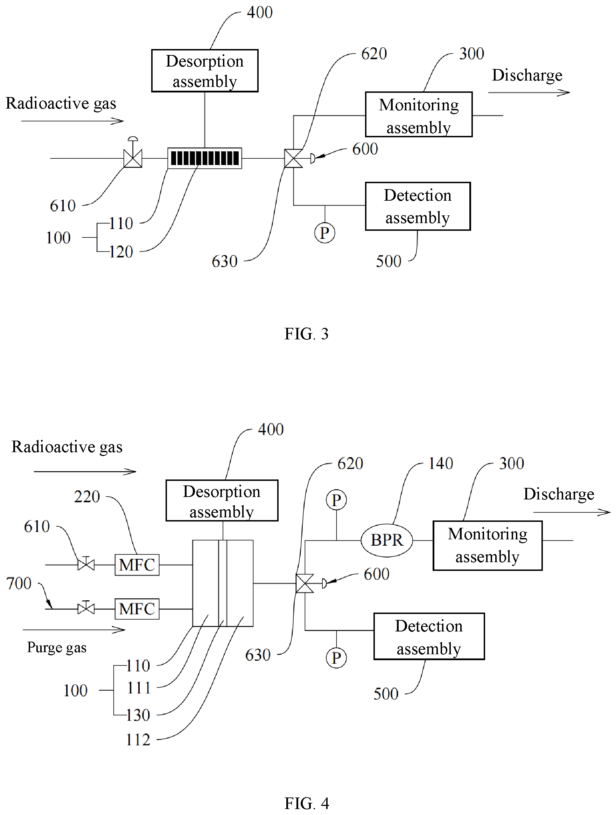

Referring to FIG. 4, FIG. 5, and FIG. 6, in some embodiments of the present disclosure, the radioactive gas detection device further includes a transfer assembly 700 configured to transfer the target radionuclide released from the adsorption assembly 100 to the detection assembly 500. It should be noted that the transfer assembly 700 can transfer the target radionuclide released from the adsorption assembly 100 into the cavity of the detection assembly 500 by means of negative pressure transfer or purge transfer, to facilitate the subsequent detection of the target radionuclide by the detection assembly 500.

Referring to FIG. 4, FIG. 5, and FIG. 6, in some specific embodiments of the present disclosure, the transfer assembly 700 uses purge transfer, negative pressure transfer, or a combination thereof, etc.

When the transfer assembly 700 uses purge transfer, referring to FIG. 4, the transfer assembly 700, the adsorption assembly 100, and the detection assembly 500 are in communication in sequence. The transfer assembly 700 is configured to deliver a purge gas that does not react with the target radionuclide to the detection assembly 500, so as to purge the target radionuclide released from the adsorption assembly 100 to the detection assembly 500.

Specifically, the purging of the transfer assembly 700 and the heating of the adsorption assembly 100 by the desorption assembly 400 may be performed at the same time to achieve the transfer of the target radionuclide. Alternatively, the desorption assembly 400 first heats the adsorption assembly 100, and then the transfer assembly 700 performs purging. In this embodiment, the purge gas that does not react with the target radionuclide may be an inert gas or other gas that does not react with the target radionuclide. The transfer assembly 700 includes a purge gas source that continuously supplies the purge gas, thereby purging the target radionuclide released from the adsorption assembly 100 into the cavity of the detection assembly 500.

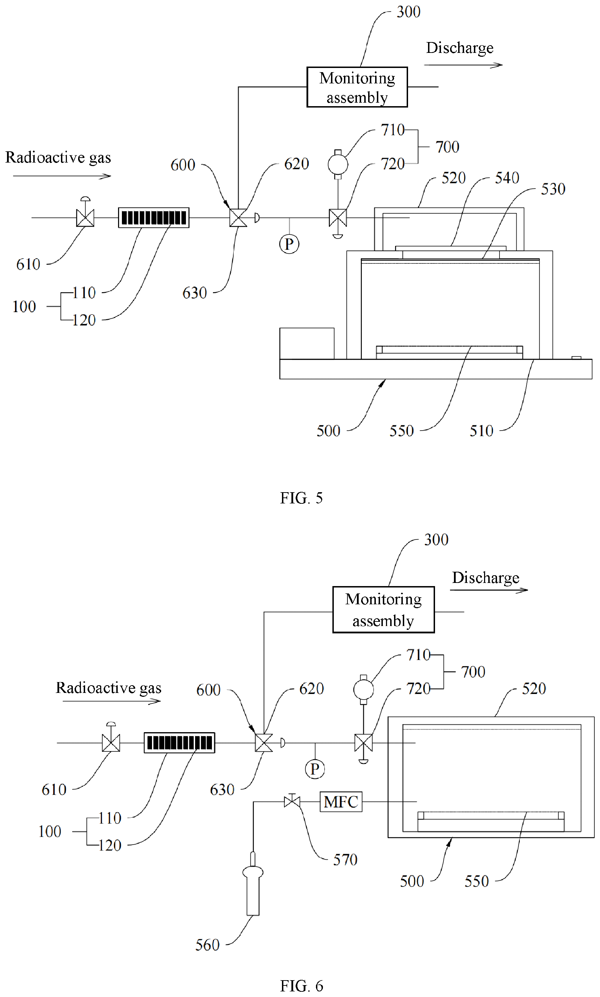

When the transfer assembly 700 uses negative pressure transfer, referring to FIG. 5 and FIG. 6, the transfer assembly 700 includes a vacuumizing component 710 and a second control valve assembly 720. The vacuumizing component 710 is in communication with the detection assembly 500, and the second control valve assembly 720 is configured to control communication between the detection assembly 500 and the vacuumizing component 710, and between the detection assembly 500 and the adsorption assembly 100. The controller is configured to: in response to the transfer assembly 700 performing a transfer operation, control the second control valve assembly 720 to communicate the detection assembly 500 with the vacuumizing component 710 and disconnect the detection assembly 500 from the adsorption assembly 100, and control the vacuumizing component 710 to perform a vacuumizing operation. The controller is further configured to: in response to a preset negative pressure being formed in the detection assembly 500, control the second control valve assembly 720 to disconnect the detection assembly 500 from the vacuumizing component 710 and communicate the detection assembly 500 with the adsorption assembly 100.

For example, before the negative pressure transfer is performed, it is necessary to form a negative pressure in the cavity of the detection assembly 500 in advance, and the target radionuclides are pre-detached from the adsorption assembly 100. In this process, the adsorption assembly 100 is disconnected from the detection assembly 500. Then, when a preset negative pressure is formed in the detection assembly 500, i.e., when the preparation is completed, the second control valve assembly 720 is controlled to communicate the adsorption assembly 100 with the detection assembly 500. Under the action of the negative pressure, the target radionuclide released from the adsorption assembly 100 will be adsorbed into the cavity of the detection assembly 500 for subsequent detection. In this embodiment, the vacuumizing component 710 is a vacuum pump, and the preset negative pressure is a set value, which may be set according to requirements, as long as the target radionuclide can be extracted into the cavity of the detection assembly 500 by the negative pressure. A vacuum gauge may be provided in the detection assembly 500 to display the negative pressure value in the detection assembly 500. The vacuum gauge may be connected to the controller by a network to facilitate automated control. The second control valve assembly 720 includes a fourth valve arranged between the adsorption assembly 100 and the detection assembly 500.

In addition, when the transfer assembly 700 performs negative pressure transfer and the detection assembly 500 is in communication with the adsorption assembly 100, a certain amount of purge gas may be continuously introduced to fully discharge the target radionuclide released from the adsorption assembly 100.

Referring to FIG. 5, it should be noted that the detection assembly 500 may be implemented as a windowed detector or a windowless detector. In the windowed detector, a partition membrane 530 is provided to cover the incidence window. When the transfer assembly 700 evacuates the detection chamber 520, the partition membrane 530 deforms toward the detection chamber 520 under the negative pressure, which may cause damage to the partition membrane 530. In addition, when the transfer assembly 700 purges the detection chamber 520, the partition membrane 530 may also be disturbed by airflow, which may also cause damage to the partition membrane 530. To avoid damage to the partition membrane 530, in some specific embodiments of the present disclosure, the detection assembly 500 includes an equipment chamber 510, a detection chamber 520, a partition membrane 530, a partition plate 540, and a detection component 550. The equipment chamber 510 and the detection chamber 520 are in communication with each other through an incidence window, and the detection chamber 520 is in communication with the adsorption assembly 100. The partition membrane 530 is arranged at the incidence window. The partition plate 540 is arranged at the incidence window and is located on a side of the partition membrane 530 away from the equipment chamber 510, and the partition plate 540 is configured to block or expose the incidence window. The detection component 550 is arranged in the equipment chamber 510. The controller is configured to: control the partition plate 540 to block the incidence window, in response to the transfer assembly 700 transferring the target radionuclide released from the adsorption assembly 100 to the detection chamber 520. The controller is further configured to: control the partition plate 540 to expose the incidence window and control the detection component 550 in the equipment chamber 510 to perform the detection operation, in response to a transfer operation having been completed for the adsorption assembly 100.

For example, referring to FIG. 5, the detection assembly 500 is implemented as a windowed detector, the detection component 550 is arranged in the equipment chamber 510, and the target radionuclide is located in the detection chamber 520. When the transfer assembly 700 evacuates the detection chamber 520 or purges the target radionuclide released from the adsorption assembly 100, the partition plate 540 blocks the incidence window to reduce the impact of the transfer assembly 700 on the partition membrane 530. In addition, after the transfer assembly 700 stops evacuating and the detection chamber 520 is in communication with the adsorption assembly 100, the partition plate 540 exposes the incident window, allowing the detection component 550 to perform the detection operation. Alternatively, when the transfer assembly 700 stops purging, the partition plate 540 exposes the incidence window, allowing the detection component 550 to perform the detection operation. In this embodiment, the target radionuclide continuously decays. During the decay process, the target radionuclide emits particles in all directions. Some of these particles are emitted toward the detection component 550, either perpendicularly or obliquely. When the target radionuclide is dispersed in the detection chamber 520, the particles can directly enter the equipment chamber 510 through the incidence window and be detected by the detection component 550. The detection component 550 can detect the particles emitted in different directions in the detection chamber 520. As such, more particles can be detected, thereby improving the detection accuracy of the detection component 550. The detection chamber 520 is a closed chamber, and an inner peripheral wall of the detection chamber 520 is provided with a reflective layer that easily reflect particles. The equipment chamber 510 is also a closed chamber. The detection component 550 is a microstructure gas detector. The partition membrane 530 is a particle-permeable film, such as a PT film. The transfer assembly 700 further includes a driving source. The driving source is operatively connected to the partition plate 540 to drive the partition plate 540 to block or expose the incidence window. The driving source may be a motor-driven telescopic structure or telescopic rod or a motor to drive the partition plate 540 to rotate. After moving, the partition plate 540 may be located on one side of the incidence window or may be located on a bottom surface of the incidence window at the detection chamber 520, and the partition plate 540 is rotated to a side surface of the detection chamber 520. The driving source is in signal connection with the controller. The controller controls the driving source to drive the partition plate 540 to block or expose the incidence window. In addition, the partition plate 540 may expose only a part of the incidence window.

It should be understood that by detecting the target radionuclide in the separate detection chamber 520, the detection component 550 can detect more particles, thereby improving the detection accuracy of the detection component 550. In addition, the partition plate 540 is used to cover the incidence window, the partition plate 540 is located on the side of the partition membrane 530 away from the detection chamber 520. In this way, when the transfer assembly 700 performs vacuuming or purging operations, the partition plate 540 supports the partition membrane 530 or blocks the incidence window, thereby protecting the partition membrane 530 and minimizing damage to the partition membrane 530.

Referring to FIG. 6, in some specific embodiments of the present disclosure, the detection assembly 500 includes a detection chamber 520, a detection component 550, a working component 560, and a working valve 570. The detection chamber 520 is in communication with the adsorption assembly 100. The detection component 550 is arranged in the detection chamber 520. The working component 560 is in communication with the detection chamber 520 and is configured to introduce a working gas into the detection chamber 520. The working valve 570 is configured to control communication between the working component 560 and the detection chamber 520. The controller is configured to: in response to the transfer assembly 700 performing a transfer operation or in response to a transfer operation having been completed for the adsorption assembly 100, control the working valve 570 to communicate the working component 560 with the detection chamber 520, control the working component 560 to introduce the working gas into the detection chamber 520, and control the detection component 550 in the detection chamber 520 to perform the detection operation.

Specifically, referring to FIG. 6, the detection assembly 500 is implemented as a windowless detector, and the detection component 550 is directly arranged in the detection chamber 520 to detect the target radionuclide in the detection chamber 520. After the working gas is introduced into the detection chamber 520, the detection component 550 detects the target radionuclide. In this embodiment, the detection chamber 520 is a closed chamber, the detection component 550 is a microstructure gas detector, and the working component 560 includes a working gas source that supplies the working gas to the detection chamber 520.

Referring to FIG. 1 and FIG. 2, in some specific embodiments of the present disclosure, the sampling assembly 200 includes a sampling source, which may be a gas container 210 that stores the radioactive gas or a to-be-tested pipeline 230 that discharges the radioactive gas. The gas container 210 serves as an object for collecting the radioactive gas, such as a storage tank, or a storage bag, etc. The to-be-tested pipeline 230 is a pipeline, such as a chimney, an exhaust vent pipe, or other space to be measured, from which the radioactive gas is being exhausted in real time.

When the sampling source is a gas container 210 that stores the radioactive gas, referring to FIG. 1, the sampling assembly 200 includes a gas container 210 and a gas flow meter 220. The gas container 210 is in communication with the adsorption assembly 100 and is configured to store the radioactive gas. The gas flow meter 220 is arranged in a pipeline between the gas container 210 and the adsorption assembly 100 and is configured to measure a total amount of the radioactive gas entering the adsorption assembly 100. The gas flow meter 220 is a mass flow controller (MFC).

Specifically, the radioactive gas stored in the gas container 210 is introduced into the adsorption assembly 100, allowing adsorption of the target radionuclide from the radioactive gas. The gas flow meter 220 detects the total amount of the radioactive gas delivered from the gas container 210. When the monitoring assembly 300 detects that the target radionuclide is contained in the radioactive gas that has passed through the adsorption assembly 100, the delivery of the gas container 210 is ended, and the total amount of the radioactive gas detected by the gas flow meter 220 is recorded. It should be noted that some target radionuclides have long half-lives, allowing for extended detection durations. Such target radionuclides are suitable for detection using the gas container 210, which provides higher detection accuracy. In addition, the radioactive gas that has passed through the monitoring assembly 300 may be directly discharged or collected again.

When the sampling source is implemented as a to-be-tested pipeline 230 that discharges the radioactive gas, referring to FIG. 2, the sampling assembly 200 includes a to-be-tested pipeline 230, a sample inlet pipeline 240, a sample outlet pipeline 250, and a gas flow meter 220. The to-be-tested pipeline 230 is configured to discharge the radioactive gas and has a first position and a second position distributed in sequence along a discharge direction. The sample inlet pipeline 240 is in communication with the to-be-tested pipeline 230 and the adsorption assembly 100 when the to-be-tested pipeline 230 is at the first position. The sample outlet pipeline 250 is in communication with the to-be-tested pipeline 230 and the monitoring assembly 300 when the to-be-tested pipeline 230 is at the second position. The gas flow meter 220 is arranged in the sample inlet pipeline 240 and is configured to measure a total amount of the radioactive gas entering the adsorption assembly 100.

Specifically, the radioactive gas flowing in the to-be-tested pipeline 230 is introduced into the adsorption assembly 100 via the sample inlet pipeline, allowing adsorption of the target radionuclide from the radioactive gas. The gas flow meter 220 detects the total amount of the radioactive gas delivered from the gas container 210. When the monitoring assembly 300 detects that the target radionuclide is contained in the radioactive gas that has passed through the adsorption assembly 100, the sampling from the sample inlet pipeline is ended, and the total amount of the radioactive gas detected by the gas flow meter 220 is recorded. Real-time sampling is performed in the to-be-tested pipeline 230 through the sample inlet pipeline 240. The sample outlet pipeline 250 discharges the radioactive gas from which the target radionuclide has been adsorbed into the to-be-tested pipeline 230 again, and the sample outlet pipeline 250 discharges the radioactive gas when the to-be-tested pipeline 230 is at the second position, so as not to affect the radioactive gas in the sample inlet pipeline 240. It should be noted that some target radionuclides have short half-lives, allowing for short detection durations. If detection were performed using the gas container 210, the target radionuclides might have completely decayed during the transportation of the gas container 210, in which case no target radionuclide could be detected. Therefore, sampling and detection are directly performed during the discharge of the target radionuclide, thereby improving the detection accuracy of the target radionuclide. Certainly, the method of directly sampling from the to-be-tested pipeline 230 that discharges the radioactive gas is also applicable to scenarios where the target radionuclides have long half-lives.

Referring to FIG. 3, in some specific embodiments of the present disclosure, the adsorption assembly 100 includes a housing 110 and a porous adsorption member 120. An adsorption chamber is defined by the housing 110. The adsorption chamber has a first communication position and a second communication position. The housing 110 is in communication with the sampling assembly 200 when the adsorption chamber is at the first communication position, the housing 110 is in communication with the monitoring assembly 300 or the detection assembly 500 when the adsorption chamber is at the second communication position. The porous adsorption member 120 is arranged in the adsorption chamber and is arranged between the first communication position and the second communication position along a delivery direction of the radioactive gas.

The housing 110 is made of stainless steel, and its specific shape and size can be selected according to requirements. The porous adsorption member 120 includes granular and/or powdered materials such as metal-organic frameworks (MOFs), covalent organic frameworks (COFs), zeolites, molecular sieves, and activated carbon derivatives. In this embodiment, the housing 110 is in the shape of a hollow cylinder. A granular or powdered material is sequentially filled into the housing 110 along an axial direction of the cylinder to form the porous adsorption member 120. The housing 110 is provided with a first communication position and a second communication position respectively at two ends of the housing 110 along the axial direction of the cylinder. The radioactive gas will pass through the porous adsorption member 120 along the axial direction of the cylinder and fully contact the porous adsorption member 120, so that the target radionuclides are embedded in the pores of the porous adsorption member 120.

Referring to FIG. 4, in some other specific embodiments of the present disclosure, the adsorption assembly 100 includes a housing 110 and a separation membrane 130. A first chamber 111 and a second chamber 112 are defined by the housing 110. The first chamber is in communication with the sampling assembly 200, the second chamber is in communication with the monitoring assembly 300 or the detection assembly 500. The separation membrane 130 is arranged between the first chamber 111 and the second chamber 112 and is in communication with the first chamber 111 and the second chamber 112.

The housing 110 is also made of stainless steel, and its shape and size can be selected according to requirements. The separation membrane 130 is implemented as a film made of a material such as MOF or COF. In this embodiment, the housing 110 is also in the shape of a hollow cylinder. The separation membrane 130 is arranged in the middle of the hollow cylinder along an axial direction of the hollow cylinder, thereby dividing the hollow cylinder into a first chamber 111 and a second chamber 112 which are substantially identical. Certainly, the position of the separation membrane 130 can also be arranged according to requirements. For example, the separation membrane 130 may be arranged at one-third of an axial length of the hollow cylinder, thereby dividing the hollow cylinder into a first chamber 111 with one-third of the size of the cylinder and a second chamber 112 with two-thirds of the size of the cylinder, or into a second chamber 112 with one-third of the size of the cylinder and a first chamber 111 with two-thirds of the size of the cylinder.

In addition, the desorption assembly 400 heats the porous adsorbent material. For example, the desorption assembly 400 may heat the housing 110 to indirectly heat the porous adsorption member 120 or the separation membrane 130. Alternatively, a heating portion of the desorption assembly 400 may be arranged in the housing 110 to directly heat the porous adsorption member 120 or the separation membrane 130. The radioactive gas detection device further includes an activation assembly configured to activate the porous adsorption member 120 or the separation membrane 130, thereby improving the adsorption effect of the porous adsorption member 120 or the separation membrane 130 for the target radionuclide. The activation assembly is configured to introduce an activation gas, such as nitrogen, into the adsorption assembly 100. After activation for a certain period of time, the activation assembly can remove impurities and moisture in the porous adsorption member 120 or the separation membrane 130, thereby improving the adsorption effect.

Referring to FIG. 4, in some specific embodiments of the present disclosure, the adsorption assembly 100 includes a housing 110 and a separation membrane 130, and a pressure in the second chamber 112 is lower than a pressure in the first chamber 111.

It should be noted that the pressure in the second chamber 112 is controlled to be lower than the pressure in the first chamber 111, so that the radioactive gas in the first chamber 111 continuously moves toward the second chamber 112. As a result, the target radionuclides are continuously embedded in the separation membrane 130, thereby improving the embedding efficiency of the target radionuclides.

In this embodiment, the radioactive gas detection device further includes a back pressure valve 140 arranged between the adsorption assembly 100 and the monitoring assembly 300. The back pressure valve 140 is configured to adjust the pressure in the second chamber 112 to be lower than the pressure in the first chamber 111 when the pressure in the second chamber 112 is a preset pressure. Specifically, after the radioactive gas is introduced for a period of time, both the first chamber 111 and the second chamber 112 are filled up with the radioactive gas, and the pressure in the first chamber 111 and the pressure in the second chamber 112 are substantially the same and continue to increase as additional radioactive gas is introduce. However, when the pressure in the first chamber 111 and the pressure in the second chamber 112 are substantially the same, the efficiency with which the target radionuclides are embedded into the separation membrane 130 decreases.. Therefore, when the pressure in the second chamber 112 increases to the preset pressure which is higher than external pressure, the back pressure valve 140 is opened due to the pressure difference. This allows the radioactive gas in the second chamber 112 to be discharged. The pressure in the second chamber 112 decreases to a level lower than the pressure in the first chamber 111, thereby improving the efficiency with which the target radionuclides are embedded into the separation membrane 130.

According to a second aspect of the present disclosure, an embodiment provides a detection method, including:
S1: delivering a radioactive gas to pass through an adsorption assembly 100, allowing the adsorption assembly 100 to adsorb a target radionuclide from the radioactive gas;
S2: in response to a monitoring assembly 300 detecting that the target radionuclide is contained in the radioactive gas that has passed through the adsorption assembly 100, ending the delivery of the radioactive gas and acquiring a total amount X₁ of the delivered radioactive gas and a content X₂ of the target radionuclide adsorbed by the adsorption assembly 100; and
S3: acquiring a concentration of the target radionuclide according to the total amount X₁ of the radioactive gas and the content X₂ of the target radionuclide.

Specifically, when the radioactive gas is delivered to the adsorption assembly 100, the total amount of the delivered radioactive gas is calculated in real time. For example, a gas flow meter 220 is provided at a position upstream of the adsorption assembly 100. The gas flow meter 220 calculates the amount of the radioactive gas passing therethrough in real time. When the monitoring assembly 300 detects that the target radionuclide is contained in the radioactive gas that has passed through the adsorption assembly 100, the adsorption is ended, and the total amount X₁ of the delivered radioactive gas is calculated. Then, the adsorption assembly 100 releases the adsorbed target radionuclide, and the detection assembly 500 detects the target radionuclide released from the adsorption assembly 100 to obtain the content X₂ of the target radionuclide. The content X₂ of the target radionuclide is divided by the total amount X₁ of the radioactive gas to obtain the concentration of the target radionuclide in the radioactive gas. It is determined whether the concentration of the target radionuclide meets an emission requirement.

It should be noted that, by using the monitoring assembly 300 to monitor whether the radioactive gas that has passed through the adsorption assembly 100 contains the target radionuclide, it can be determined whether the adsorption assembly 100 has completed adsorption. This enables the adsorption assembly 100 to adsorb a sufficient amount of target radionuclides for detection, while allowing fewer target radionuclides to pass through the monitoring assembly 300. As a result, the detection accuracy and the content accuracy of the target radionuclide are improved, thereby enhancing the detection accuracy of the concentration of the target radionuclide in the radioactive gas.

In some specific embodiments of the present disclosure, in S2, heating is performed to release the target radionuclide adsorbed by the adsorption assembly 100, and the content X₂ of the target radionuclide released from the adsorption assembly 100 is measure, where a temperature of the heating ranges from 80°C to 100°C.

It should be understood that the heating operation causes the porous material in the adsorption assembly 100 to thermally expand, thereby allowing the target radionuclides to be detached from the pores of the porous material. This allows separation of the target radionuclides from the porous material for detection. In this embodiment, the heating temperature is controlled to range from 80°C to 100°C to reduce energy consumption and detection costs, and the heating time is controlled to range from 1 hour to 2 hours. Certainly, the specific time can be extended or shortened according to the amount of the porous material.

The embodiments of the present disclosure have been described in detail above with reference to the accompanying drawings, but the present disclosure is not limited to the above embodiments, and various changes may be made within the knowledge of those having ordinary skills in the art without departing from the gist of the present disclosure.

## Claims

1. A radioactive gas detection device for detecting a radioactive gas containing a target radionuclide, comprising:
an adsorption assembly, configured to adsorb the target radionuclide in the radioactive gas;
a sampling assembly, in communication with the adsorption assembly and configured to supply the radioactive gas to the adsorption assembly;
a monitoring assembly, in communication with the adsorption assembly and configured to monitor the target radionuclide in the radioactive gas that has passed through the adsorption assembly;
a desorption assembly, configured to cause the adsorption assembly to release the adsorbed target radionuclide;
a detection assembly, in communication with the adsorption assembly and configured to measure a content of the target radionuclide released from the adsorption assembly;
a first control valve assembly, configured to control communication between the adsorption assembly and the sampling assembly, between the adsorption assembly and the monitoring assembly, and between the adsorption assembly and the detection assembly; and
a controller,
wherein, the controller is configured to: in response to the monitoring assembly detecting that the target radionuclide is contained in the radioactive gas that has passed through the adsorption assembly, control the first control valve assembly to disconnect the adsorption assembly from the sampling assembly, disconnect the adsorption assembly from the monitoring assembly, and communicate the adsorption assembly with the detection assembly, and control the detection assembly to perform a detection operation on the target radionuclide released from the adsorption assembly to obtain the content of the target radionuclide.

2. The radioactive gas detection device of claim 1, wherein the radioactive gas detection device further comprises a transfer assembly configured to transfer the target radionuclide released from the adsorption assembly to the detection assembly.

3. The radioactive gas detection device of claim 2, wherein:
the detection assembly comprises: an equipment chamber, a detection chamber, a partition membrane, a partition plate, and a detection component, wherein the equipment chamber and the detection chamber are in communication with each other through an incidence window, and the detection chamber is in communication with the adsorption assembly; the partition membrane is arranged at the incidence window; the partition plate is arranged at the incidence window and is located on a side of the partition membrane away from the equipment chamber, and the partition plate is configured to block or expose the incidence window; the detection component is arranged in the equipment chamber; and
the controller is configured to: control the partition plate to block the incidence window, in response to the transfer assembly transferring the target radionuclide released from the adsorption assembly to the detection chamber; and control the partition plate to expose the incidence window and control the detection component in the equipment chamber to perform the detection operation, in response to a transfer operation having been completed for the adsorption assembly.

4. The radioactive gas detection device of claim 2, wherein the detection assembly comprises: a detection chamber, a detection component, a working component, and a working valve, wherein the detection chamber is in communication with the adsorption assembly, the detection component is arranged in the detection chamber, the working component is in communication with the detection chamber and is configured to introduce a working gas into the detection chamber, and the working valve is configured to control communication between the working component and the detection chamber; and
the controller is configured to: in response to the transfer assembly performing a transfer operation or in response to a transfer operation having been completed for the adsorption assembly, control the working valve to communicate the working component with the detection chamber, control the working component to introduce the working gas into the detection chamber, and control the detection component in the detection chamber to perform the detection operation.

5. The radioactive gas detection device of claim 2, wherein the transfer assembly, the adsorption assembly, and the detection assembly are in communication in sequence, and the transfer assembly is configured to deliver a purge gas that does not react with the target radionuclide to the detection assembly to purge the target radionuclide released from the adsorption assembly to the detection assembly; and/or
the transfer assembly comprises a vacuumizing component and a second control valve assembly, the vacuumizing component is in communication with the detection assembly, and the second control valve assembly is configured to control communication between the detection assembly and the vacuumizing component and communication between the detection assembly and the adsorption assembly; and
wherein the controller is configured to: in response to the transfer assembly performing a transfer operation, control the second control valve assembly to communicate the detection assembly with the vacuumizing component and disconnect the detection assembly from the adsorption assembly, and control the vacuumizing component to perform a vacuumizing operation; and in response to a preset negative pressure being formed in the detection assembly, control the second control valve assembly to disconnect the detection assembly from the vacuumizing component and communicate the detection assembly with the adsorption assembly.

6. The radioactive gas detection device of claim 1, wherein
the sampling assembly comprises a gas container and a gas flow meter, the gas container is in communication with the adsorption assembly and is configured to store the radioactive gas, and the gas flow meter is arranged in a pipeline between the gas container and the adsorption assembly and is configured to measure a total amount of the radioactive gas entering the adsorption assembly; or
the sampling assembly comprises a to-be-tested pipeline, a sample inlet pipeline, a sample outlet pipeline, and a gas flow meter, the to-be-tested pipeline is configured to discharge the radioactive gas and has a first position and a second position distributed in sequence along a discharge direction, the sample inlet pipeline is in communication with the to-be-tested pipeline and the adsorption assembly when the to-be-tested pipeline is at the first position, the sample outlet pipeline is in communication with the to-be-tested pipeline and the monitoring assembly when the to-be-tested pipeline is at the second position, and the gas flow meter is arranged in the sample inlet pipeline and is configured to measure a total amount of the radioactive gas entering the adsorption assembly.

7. The radioactive gas detection device of claim 1, wherein
the adsorption assembly comprises a housing and a porous adsorption member, an adsorption chamber is defined by the housing, the adsorption chamber has a first communication position and a second communication position, the housing is in communication with the sampling assembly when the adsorption chamber is at the first communication position, the housing is in communication with the monitoring assembly or the detection assembly when the adsorption chamber is at the second communication position, and the porous adsorption member is arranged in the adsorption chamber and is arranged between the first communication position and the second communication position along a delivery direction of the radioactive gas;
or
the adsorption assembly comprises a housing and a separation membrane, a first chamber and a second chamber are defined by the housing, the first chamber is in communication with the sampling assembly, the second chamber is in communication with the monitoring assembly or the detection assembly, and the separation membrane is arranged between the first chamber and the second chamber and is in communication with the first chamber and the second chamber.

8. The radioactive gas detection device of claim 7, wherein the adsorption assembly comprises a housing and a separation membrane, and a pressure in the second chamber is lower than a pressure in the first chamber.

9. A detection method, comprising:
S1: delivering a radioactive gas to pass through an adsorption assembly, allowing the adsorption assembly to adsorb a target radionuclide from the radioactive gas;
S2: in response to a monitoring assembly detecting that the target radionuclide is contained in the radioactive gas that has passed through the adsorption assembly, ending the delivery of the radioactive gas and acquiring a total amount X₁ of the delivered radioactive gas and a content X₂ of the target radionuclide adsorbed by the adsorption assembly; and
S3: acquiring a concentration of the target radionuclide according to the total amount X₁ of the radioactive gas and the content X₂ of the target radionuclide.

10. The detection method of claim 9, wherein: in S2, heating is performed to release the target radionuclide adsorbed by the adsorption assembly, and the content X₂ of the target radionuclide released from the adsorption assembly is measured,
wherein a temperature of the heating ranges from 80°C to 100°C.
